(19) **Europäisches Patentamt European Patent Office Office européen des brevets**

(11) **EP 4 467 655 A1**

(12) **EUROPEAN PATENT APPLICATION**
published in accordance with Art. 153(4) EPC

(43) Date of publication:
**27.11.2024 Bulletin 2024/48**

(21) Application number: **23742796.8**

(22) Date of filing: **13.01.2023**

(51) International Patent Classification (IPC):
**C12Q 1/02** (2006.01)   **C12Q 1/46** (2006.01)
**A61K 35/12** (2015.01)

(86) International application number:
**PCT/CN2023/071987**

(87) International publication number:
**WO 2023/138481 (27.07.2023 Gazette 2023/30)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.01.2022 CN 202210059880**

(71) Applicant: **Academy of Military Medical Sciences
Beijing 100850 (CN)**

(72) Inventors:
• **MA, Bo
Beijing 100850 (CN)**

• **WANG, Lili
Beijing 100850 (CN)**
• **LI, Zhi
Beijing 100850 (CN)**
• **GUO, Lei
Beijing 100850 (CN)**
• **XU, Hua
Beijing 100850 (CN)**
• **XIE, Jianwei
Beijing 100850 (CN)**

(74) Representative: **Weickmann & Weickmann
PartmbB
Postfach 860 820
81635 München (DE)**

(54) **METHOD FOR SCREENING ACETYLCHOLINESTERASE INHIBITOR**

(57)    The present disclosure relates to a method for screening an acetylcholinesterase inhibitor, and further relates to a kit and system for screening an acetylcholinesterase inhibitor.

**EP 4 467 655 A1**

Processed by Luminess, 75001 PARIS (FR)

## Description

The present application is based on and claims the benefits of priorities from Chinese application No. 202210059880.4 filed on January 19, 2022, the disclosures of which are incorporated herein by reference in their entirety.

## Technical Field

The present disclosure belongs to the fields of food, environment, pharmacology and medicine, and specifically relates to a method for screening acetylcholinesterase inhibitors, and also relates to a kit and system for screening acetylcholinesterase inhibitors.

## Background Art

Acetylcholinesterase (AChE), also known as true cholinesterase, is a key enzyme in the biological nerve conduction process. AChE can degrade acetylcholine (ACh) at cholinergic synapses, terminate the excitatory action of neurotransmitters on the postsynaptic membrane, and ensure the normal transmission of nerve signals in the body. Currently known organophosphates (OPs) neurotoxic substances, carbamate (CM) pesticides, and Alzheimer's disease (AD) therapeutic drugs all use AChE as an important target, and they induce neurotoxic effects or improve cognitive function in AD by inhibiting normal or pathologically elevated AChE activity. OPs are the largest class of AChE inhibitors (AChEIs), and related compounds, including pesticides, defoliants, flame retardants, industrial solvents, lubricants, plasticizers, fuel additives, and neurotoxic chemical warfare agents, as well as AD prescription drug metrifonate, are currently found in hundreds of products used around the world. Due to the great advantages of pesticides in improving agricultural productivity and controlling deadly vector-borne diseases, and with the rapid development of industrialization, OPs are extensively utilized. According to statistics, approximately 1 billion pounds of OPs are released into the environment, food, and water supplies globally every year, making OPs one of the most common synthetic chemicals detected in the environment, animal tissues, and human tissues. This has brought about increasingly serious environmental and ecological problems, posing a huge threat to human health and public safety. In addition, in the face of the increasingly severe problem of the aging of the global population and the far unmet need for AD treatment, as confirmed AD therapeutic drugs, new type (non-organophosphorus) AChEIs are still the focus of AD drug research and development. Therefore, the screening and accurate characterization of known and potential AChEIs (including organophosphorus nerve agents) are important for the early warning of environment and food safety, understanding of potentially toxic substances, accurate treatment of poisoning, and research on new and more efficient AD drugs.

So far, there are three main types of commonly used AChEI screening methods, namely, determination based on known OP structure, analysis based on inhibition of AChE activity, and immunoassay based on OP immunogenicity. Due to the limitations in preparing antibodies with strong affinity and high specificity, immunoassay methods not only face challenges in successfully detecting low-level OPs, but are also unable to distinguish OPs with different structures. Analysis based on chemical structure mainly uses chromatographic methods, such as liquid chromatography (LC), liquid chromatography-mass spectrometry (LC-MS), liquid chromatography-tandem mass spectrometry (LC-MS/MS), gas chromatography (GC), and gas chromatography-mass spectrometry (GC-MS). These methods offer high sensitivity with detection limits within the nanomolar concentration range, and are the preferred method for trace quantitative analysis. However, these method are only suitable for known molecules, have low throughput and is not suitable for large-scale screening. In addition, they are time-consuming, uses expensive equipment, and must be performed by well-trained technicians. The enzyme activity inhibition method is based on the inhibition of AChE, and AChEI can be detected by measuring the enzyme activity before and after exposure to the sample to be tested. Enzyme activity is usually measured by Ellman colorimetric analysis, radioactive analysis, fluorescence analysis, electrochemical analysis, or chemiluminescence analysis. These methods are low cost, have high throughput, and are fast in analysis, and are the most promising alternatives to classic methods (GC/MS, LC/MS). However, these methods are insensitive to changes in enzyme activity after exposure to some OPs compounds, which severely limits the detection sensitivity of these methods. In addition, from the perspective of efficient screening of AChEI, the above methods can only provide a single characterization of the chemical to be tested at the level of known structures or enzyme molecules, and cannot provide biological characterization of potentially toxic molecules, nor provide multidimensional information for accurate early warning and efficient treatment guidance.

## Contents of the Invention

The present disclosure relates to a method for screening an acetylcholinesterase inhibitor, comprising:

1) mixing a sample to be screened with an acetylcholinesterase;

2) mixing the mixed system obtained in step 1) with an acetylcholine;

3) contacting a reporter cell M3:NFATc1-EGFP U2OS with the mixed system obtained in step 2);

4) detecting the degree of activation of the reporter cell M3:NFATc1-EGFP U2OS.

In some embodiments, the degree of activation of the reporter cell M3:NFATc1-EGFP U2OS is expressed as the degree of translocation of NFATc1-EGFP from cytoplasm to nucleus in the reporter cell. If the NFATc1-EGFP in the reporter cell is translocated from cytoplasm to nucleus, it is judged that the sample to be screened contains an acetylcholinesterase inhibitor. The inhibitory activity of acetylcholinesterase inhibitor against acetylcholinesterase can also be quantified according to the number of intracellular NFATc1-EGFP translocated from cytoplasm to nucleus.

In some embodiments, the sample to be screened according to the present disclosure can be a chemical, a food (e.g., various meats, vegetables, tea, etc.), an organophosphorus pesticide in the environment, an AD therapeutic drug, a nerve agent, etc.

In some embodiments, the acetylcholine is an acetylcholine iodide, and the acetylcholinesterase is a recombinant human acetylcholinesterase.

In some embodiments, in step 1) of the method for screening acetylcholinesterase inhibitor as described in the present disclosure, the sample to be screened is mixed with the acetylcholinesterase in a first solvent.

In some embodiments, in step 2) of the method for screening acetylcholinesterase inhibitor as described in the present disclosure, the mixed system obtained in step 1) is mixed with the acetylcholine in a second solvent.

In some embodiments, the first solvent and the second solvent are the same or different, and each is independently a medium, such as DMEM high-glucose medium, suitable for the normal growth of the reporter cell M3:NFATc1-EGFP U2OS.

In some embodiments, in step 2) of the method for screening acetylcholinesterase inhibitor as described in the present disclosure, after the mixed system obtained in step 1) is mixed with the acetylcholine, the initial concentration (the concentration when it is not reacted with the acetylcholine) of the acetylcholinesterase in the obtained mixed system is 0.005 to 0.018 U/mL, preferably 0.007 to 0.015 U/mL, and further preferably 0.01 U/mL.

In some embodiments, in step 2) of the method for screening acetylcholinesterase inhibitor as described in the present disclosure, after the mixed system obtained in step 1) is mixed with the acetylcholine, the initial concentration (the concentration when it is not reacted with the acetylcholinesterase) of the acetylcholine in the obtained mixed system is 10 to 1000 nM, preferably 30 to 800 nM, further preferably 30 to 500 nM, further preferably 30 to 400 nM, further preferably 30 to 300 nM, further preferably 30 to 200 nM, further preferably 30 to 100 nM.

In some embodiments, in step 1) of the method for screening acetylcholinesterase inhibitor as described in the present disclosure, the sample to be screened is mixed with the acetylcholinesterase at 25 to 40°C (e.g., 30°C, 32°C, 35°C, 37°C) for 10 to 60 minutes, such as 20 minutes, 25 minutes, 30 minutes, 35 minutes, 40 minutes, 50 minutes.

In some embodiments, in step 2) of the method for screening acetylcholinesterase inhibitor as described in the present disclosure, the mixed system obtained in step 1) is mixed with the acetylcholine at 25 to 40°C (e.g., 30°C, 32°C, 35°C, 37°C) for 10 to 60 minutes, such as 20 minutes, 25 minutes, 30 minutes, 35 minutes, 40 minutes, 50 minutes.

In some embodiments, in step 2) of the method for screening acetylcholinesterase inhibitor as described in the present disclosure, after the mixed system obtained in step 1) is mixed with the acetylcholine, the initial concentration (the concentration when it is not reacted with the acetylcholine) of the acetylcholinesterase in the obtained mixed system is 0.005 to 0.018 U/mL, and the initial concentration of the acetylcholine in the obtained mixed system is 10 to 1000 nM (e.g., 30 to 800 nM, 30 to 500 nM, 30 to 400 nM, 30 to 300 nM, 30 to 200 nM, or 30 to 100 nM).

In some embodiments, in step 2) of the method for screening acetylcholinesterase inhibitor as described in the present disclosure, after the mixed system obtained in step 1) is mixed with the acetylcholine, the initial concentration (the concentration when it is not reacted with the acetylcholine) of the acetylcholinesterase in the obtained mixed system is 0.007 to 0.015 U/mL, and the initial concentration of the acetylcholine in the obtained mixed system is 10 to 1000 nM (e.g., 30 to 800 nM, 30 to 500 nM, 30 to 400 nM, 30 to 300 nM, 30 to 200 nM, or 30 to 100 nM).

In some embodiments, in step 2) of the method for screening acetylcholinesterase inhibitor as described in the present disclosure, after the mixed system obtained in step 1) is mixed with the acetylcholine, the initial concentration (the concentration when it is not reacted with the acetylcholine) of the acetylcholinesterase in the obtained mixed system is 0.01 U/mL, and the initial concentration of the acetylcholine in the obtained mixed system is 10 to 1000 nM (e.g., 30 to 800 nM, 30 to 500 nM, 30 to 400 nM, 30 to 300 nM, 30 to 200 nM, or 30 to 100 nM).

In some embodiments, in step 2) of the method for screening acetylcholinesterase inhibitor as described in the present disclosure, after the mixed system obtained in step 1) is mixed with the acetylcholine, the initial concentration of the acetylcholinesterase in the obtained mixed system is 0.01 U/mL, and the initial concentration of the acetylcholine in the obtained mixed system is 30 nM, preferably, the mixed system obtained in step 1) is mixed with the acetylcholine for 10 minutes.

In some embodiments, in step 2) of the method for screening acetylcholinesterase inhibitor as described in the present disclosure, after the mixed system obtained in step 1) is mixed with the acetylcholine, the initial concentration of the acetylcholinesterase in the obtained mixed system is 0.01 U/mL, and the initial concentration of the acetylcholine in the obtained mixed system is 100 nM, preferably, the mixed system obtained in step 1) is mixed with the acetylcholine for 20 minutes.

In some embodiments, in step 3) of the method for screening acetylcholinesterase inhibitor as described in the present disclosure, the reporter cell M3:NFATc1-EGFP U2OS is contacted with the mixed system obtained in step 2) for 10 to 60 minutes, for example, 20 minutes, 25 minutes, 30 minutes, 35 minutes, 40 minutes, 50 minutes.

In some embodiments, before step 4) of the method for screening acetylcholinesterase inhibitor as described in the present disclosure, the method further comprises:

fixing the reporter cell with a formaldehyde solution diluted in phosphate buffer (PBS);

staining (e.g., fluorescently staining) the nucleus and/or cytoskeleton (actin, tubulin) of the reporter cell.

In some embodiments, the method for screening acetylcholinesterase inhibitor as described in the present disclosure uses a high-content cell imaging and analysis system to detect the degree of activation of the reporter cell M3:NFATc1-EGFP U2OS.

The present disclosure also relates to a kit, comprising a reporter cell M3:NFATc1-EGFP U2OS, an acetylcholine (e.g., acetylcholine iodide), an acetylcholinesterase (e.g., recombinant human acetylcholinesterase), and a fluorescent dye (e.g., Hoechst 33342, phalloidin).

In some embodiments, the kit further comprises a medium, such as DMEM high glucose medium, suitable for the growth of the reporter cell M3:NFATc1-EGFP U2OS.

In some embodiments, the kit further comprises a phosphate (PBS) buffer substance (either in the form of an aqueous solution or anhydrous form), and formaldehyde.

In some embodiments, the kit further comprises an instruction describing the method for screening acetylcholinesterase inhibitor as described in the present disclosure.

The present disclosure also relates to a use of the kit for screening acetylcholinesterase inhibitor.

The present disclosure also relates to a system for screening acetylcholinesterase inhibitor, comprising the kit and a high-content cell imaging and analysis system.

The reporter cell M3:NFATc1-EGFP U2OS as described in the present disclosure is a U2OS cell stably expressing human muscarinic M3 receptor (muscarinic receptor3, M3) and human NFATc1 (Nuclear Factor Of Activated T Cells 1) fused to the C-terminus of enhanced green fluorescent protein (EGFP), when M3 is activated by the agonist ACh, intracellular $Ca^{2+}$ is released, and the increase in calcium level causes NFATc1 to be dephosphorylated and rapidly translocate from the cytoplasm to the nucleus. The cell is commercially available or can be constructed according to existing technology.

In the present disclosure, unless otherwise stated, scientific and technical terms used herein have the meanings commonly understood by those skilled in the art. Moreover, the laboratory operation steps of cell culture, molecular genetics, nucleic acid chemistry, and immunology used herein are routine operation steps widely used in the corresponding fields.

Beneficial effects of the present disclosure

In the present disclosure, a high-throughput screening method for AChE inhibitor based on ACh activity is established by using an M3 receptor-activated reporter cell as detector and optimizing the rhAChE reaction system. This method has been successfully used to screen OPs pesticides, nerve agents, and AD drug cholinesterase inhibitors, its detection limit for paraoxon is 1 nM, its detection limit for donepezil is 1 nM, its detection limit for GD (soman) is 0.1 nM, and its detection limit for VX is 30 pM. Compared with traditional methods, this method can achieve high throughput, high sensitivity, and low time and reagent consumption costs. In particular, this method can obtain phenotype spectrum information related to cytotoxicity and cellular effects of the substances to be tested in a single experiment, thereby providing richer characteristic information for the identification of potential drugs or toxic substances, and providing new and efficient tools for the screening of a large number of neurotoxic compounds.

**Brief Description of the Drawings**

Figure 1 shows the detection system of the method for screening AChEI as established in the example of the present disclosure;

Figure 2 shows that AChI activates a reporter cell line in a concentration-dependent manner and induces the nuclear translocation of EGFP-NFATc1 in M3:NFATc1-EGFP U2OS human osteosarcoma cells;

Figure 3A shows the effects of incubation of different concentrations of rhAChE with AChI for 10 min on the EGFP-NFATc1 nuclear translocation;

Figure 3B shows the effects of incubation of 0.01 U/mL rhAChE with different concentrations of AChI for different times on the EGFP-NFATc1 nuclear translocation;

Figure 3C shows the effects of VX on the EGFP-NFATc1 nuclear translocation in two reaction systems;

Figure 4 shows the results of detecting organophosphorus pesticide AChEI using the method for screening AChEI as established in the example of the present disclosure, wherein A represents the detection result of paraoxon; B represents the detection result of dichlorvos; and C represents the detection result of chlorpyrifos;

Figure 5 shows the results of detecting AD therapeutic drug AChEI using the method for screening AChEI as established in the example of the present disclosure, wherein A represents the detection result of donepezil; and B represents the detection result of rivastigmine;

Figure 6 shows the results of detecting nerve agent AChEI using the method for screening AChEI as established in the example of the present disclosure, wherein A represents the detection result of tabun; B represents the detection result of sarin; and C represents the detection result of soman;

Figure 7 shows the characterization and analysis results of the cell phenotype spectrum of AChEI using the method for screening AChEI as established in the example of the present disclosure.

**Specific Models for Carrying Out the Invention**

The technical solutions in the embodiments of the present disclosure will be clearly and completely described below with reference to the drawings in the examples of the present disclosure. Obviously, the described examples are only some of the examples of the present disclosure, rather than all of the examples. The following description of at least one exemplary example is merely illustrative in nature and is not intended to limit the present disclosure, its application or uses. Based on the examples in the present disclosure, all other examples obtained by those of ordinary skill in the art without making creative efforts fall within the scope of protection of the present disclosure.

In all examples shown and discussed herein, any specific values should be understood as illustrative only and not as limiting. Accordingly, other examples of the exemplary examples may have different values.

The present disclosure is described in detail below through a specific example.

Unless indicated otherwise, the molecular biological experimental methods and immunological assays used in the present disclosure are carried out substantially in accordance with the methods as described in Sambrook J et al., Molecular Cloning: A Laboratory Manual (Second Edition), Cold Spring Harbor Laboratory Press, 1989, and F. M. Ausubel et al., Short Protocols in Molecular Biology, 3rd Edition, John Wiley & Sons, Inc., 1995. If the specific conditions were not indicated in the examples, the conditions should be carried out according to the conventional conditions or the conditions recommended by the manufacturer. If the manufacturer of the reagents or instruments used was not indicated, they were all conventional products that can be purchased commercially. Those skilled in the art understand that the examples describe the present disclosure by way of example and are not intended to limit the protection scope of the present disclosure.

The main drugs and reagents used in the examples of the present disclosure include: DMEM high-glucose culture medium, fetal bovine serum (FBS), G418 (geneticin, 50 mg/mL), penicillin/streptomycin mixed solution (100×), 0.05% trypsin-EDTA solution, 1×PBS phosphate buffer solution (pH = 7.4), and 10× PBS phosphate buffer solution (pH = 7.4), which were purchased from Gibco; bovine serum albumin (BSA) which was purchased from Amresco; acetylcholine iodide (AChI) which was purchased from Selleck; recombinant human acetylcholinesterase (rhAChE) which was purchased from Sigma-Aldrich; Hoechst 33342 fluorescent dye which was purchased from Invitrogen; 633-labeled phalloidin which was purchased from Solarbio; mouse anti-human $\alpha$-tubulin primary antibody which was purchased from Thermo; Alexa Flour 546-labeled donkey anti-mouse secondary antibody which was purchased from Invitrogen; paraoxon, dichlorvos, and chlorpyrifos which were purchased from Merck; donepezil and rivastigmine which were purchased from Selleck; sarin, tabun, and soman and VX which were purchased from the Institute of Chemical Defense; and other reagents which were of analytical grade reagents.

The main instrument used in the embodiments of the present disclosure is Perkin Elmer Opera Phenix™ high-content cell imaging and analysis system.

The cell lines and culture conditions used in the examples of the present disclosure: M3:NFATc1-EGFP U2OS human osteosarcoma cell line (Thermo Company) was cultured in DMEM high-glucose medium containing 10% FBS in a 37°C, 5% $CO_2$ incubator. In addition, 0.5 mg/mL G418, 100 U/mL penicillin, and 100 $\mu$g/mL streptomycin were added to the culture medium.

Preparation of solutions used in the examples of the present disclosure:

1.   Recombinant human acetylcholinesterase (rhAChE) was prepared into a 1 U/mL stock solution by using 10× PBS buffer (pH=7.4) containing 0.1% BSA, and the stock solution was diluted with serum-free culture medium into a target working solution during the experiment.

2. Acetylcholine iodide (AChI) was prepared into a 5 mM stock solution by using sterile deionized water, and the stock solution was diluted with serum-free culture medium into a target working solution during the experiment.

3. Paraoxon, dichlorvos, and chlorpyrifos were prepared into 500 $\mu$M stock solutions by using methanol, and the stock solution was diluted with serum-free medium into a target working solution during the experiment.

4. Donepezil hydrochloride (Donepezil) and Rivastigmine tartrate (Rivastigmine) were prepared into 5 mM stock solutions by using sterile deionized water, and the stock solution was diluted with serum-free medium into a target working solution during the experiment.

5. Tabun (GA), sarin (GB), soman (GD), and VX were prepared into 0.1M stock solutions by using acetonitrile, and the stock solution was diluted with serum-free medium into a target working solution during the experiment.

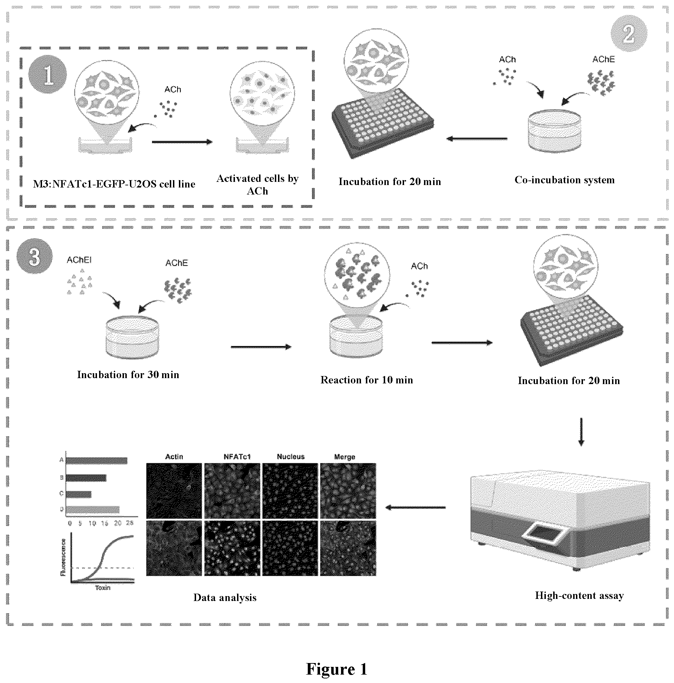

The detection system of the method for screening AChEI as established in the example of the present disclosure was shown in Figure 1, which comprised: (1) a reporter cell line based on cholinergic receptor; (2) an AChE reaction system; and, (3) a high content screening (HCS) detection system composed of (1) and (2), wherein, (1) was the cholinergic receptor cell line M3:NFATc1-EGFP U2OS, which was U2OS cell stably expressing human muscarinic choline receptor 3 (M3) and human NFATc1 fused to the C-terminal of enhanced green fluorescent protein (EGFP), when M3 was activated by the agonist ACh, intracellular $Ca^{2+}$ was released, and the increase in calcium levels caused NFATc1 to be dephosphorylated and rapidly translocate from the cytoplasm to the nucleus, and based on this, the activity of the agonist could be quantitatively analyzed; (2) was a detection system containing recombinant human AChE (rhAChE) and ACh and the reporter cell line, the content of which is determined after being optimized; and (3) used the optimized HCS detection system to detect AChEI. In the present disclosure, the acetylcholine iodide (ACh) with optimized concentration was used as an agonist; AChE was pretreated with AChEI, then ACh was added and incubated for a certain period of time, the reporter cell line was treated by using the obtained mixed system, and the HCS system was used to quantitatively detect the activity of the reporter cells after AChEI was added to the AChE reaction system, and thereby determining the inhibitory activity of AChEI against AChE and its unique cellular response characteristics.

Example 1: High-content analysis of the activation of M3:NFATc1-EGFP U2OS reporter cell line by Ach

In this example, it was examined whether ACh could stably activate M3:EGFP-NFATc1 cells. AChI was selected as Ach, a high-content cell imaging analysis system was used to collect cell images after ACh treatment of the reporter cell line, and the degree of EGFP-NFATc1 nuclear translocation was quantitatively analyzed, thereby determining the extent and concentration range of ACh activated M3 receptor. The specific steps were as follows:

A cell suspension of $1\times10^5$ cells/mL was prepared, and inoculated at 100 $\mu$L/well into a black transparent-bottom 96-well culture plate, then cultured for 24 hours, the culture supernatant was discarded, the cells were washed twice with serum-free medium, and then AChI gradient solutions (diluted in serum-free culture medium, the final concentrations were 0, 0.01, 0.03, 0.1, 0.3, 1, 3, 10, 30, 100, 300, 1000, 3000, 10000, 30000, 100000 nmol/L respectively, 3 wells for each concentration) were added at 100 $\mu$L/well respectively, and then the cells were placed in a 5% $CO_2$ incubator at 37°C for 20 minutes. Then $1 \times$ PBS solution containing 12% formaldehyde was added at 50 $\mu$L/well (the final concentration of formaldehyde was 4 %) to perform fixation at room temperature in the dark for 20 min. The cells were washed twice with $1 \times$ PBS solution, 200 $\mu$L/well of $1\times$PBS solution containing 1 $\mu$M Hoechst 33342 was added, and then the cells were incubated at 37°C in the dark for 30 min. The cells were washed twice with $1\times$PBS solution, and then 100 $\mu$L of $1\times$PBS/well was added. The cells were loaded to machine and detected, in which Alexa 488 (absorption wavelength: 495 nm, emission wavelength: 519 nm) and DAPI (absorption wavelength: 340 nm, emission wavelength: 488 nm) channels were selected, and $20\times$ objective lens was used to collect cell images, 9 visual fields per well were set. Harmony 4.9 software was used to quantitatively analyze the number of cell nuclei and intracellular NFATc1-EGFP translocation from the cytoplasm to the nucleus, the activation effect of ACh was expressed as the average degree of NFATc1-EGFP nuclear translocation in each cell for 3 replicate wells, no less than 1,000 cells per well, and the $EC_{50}$ value was calculated using Prism 8.

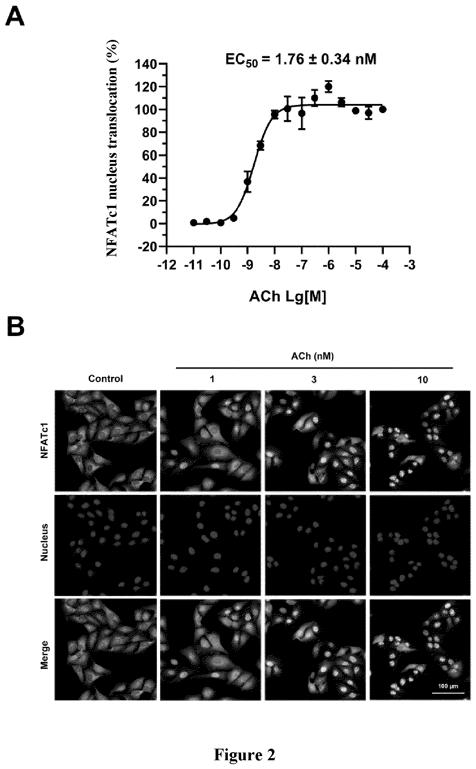

After treatment of M3:NFATc1-EGFP U2OS with different concentrations of AChI, the high-content analysis results of intracellular EGFP-NFATc1 translocation were shown in Figure 2. AChI concentration-dependently activated the reporter cell line in the range of 0.1 to 100 nM, when the concentration of AChI was 0.3 nM, the cells appeared an activation reaction; when the concentration of AChI reached 1 nM, the degree of nuclear translocation of EGFP-NFATc1 was approximately 40% the maximum degree; when the concentration of AChI reached 10 nM, the degree of nuclear translocation of EGFP-NFATc1 was close to the maximum degree; and when the concentration of AChI was 30 nM, the maximum degree of nuclear translocation of EGFP-NFATc1 was reached. Prism 8.0 software was used to nonlinearly fit log (agonist) vs. response to obtain an S-shaped curve, and then analysis showed that the $EC_{50}$ value of AChI was $1.76 \pm 0.34$ nM, and the Z' factor was 0.685, which indicated that the cholinergic receptor reporter cell analysis model using AChI as agonist was a highly sensitive and stable HTS method.

Example 2: Establishment of AChE reaction system

In this example, an AChE reaction system suitable for reporter cell line was established. The effects of different substrate (AChI) concentrations, different enzyme (rhAChE) concentrations, and different enzyme-substrate reaction times on the reaction system were investigated according to the quantitative analysis results of reporter cell line activation. The specific steps were as follows:
First, the gradient rhAChE working solutions and AChI working solutions were cross-combined, and mixed in a 96-well plate at a ratio of 1:1 (v/v), and reacted at 37°C for 5, 10, 15, and 20 minutes, respectively. Then, the mixed solution was added at 100 μL/well to a black transparent-bottom 96-well plate that was pre-inoculated with the reporter cell line, the cells were incubated for 20 minutes, then the activation degree of the reporter cell line was quantitatively analyzed according to the method described in Example 1, and the Z' factor was calculated to obtain the optimal experimental system.

The calculation formula of Z' factor was:

$$Z' = 1 - \frac{(3\sigma_{c+} + 3\sigma_{c-})}{|\mu_{c+} - \mu_{c-}|}$$

wherein, "σ" represented standard deviation; "μ" represented mean signal; "c+" represented positive control; "c-" represented negative control.

The value of Z' factor should be between 0 and 1. When the Z' factor was 0, it indicated that the experimental system was not established; when 0 < Z' factor < 0.5, it indicated that the experimental system had poor stability and was unreliable; when 0.5 ≤ Z' factor < 1, it indicated that the experimental system had good stability and good reliability; if the Z' factor was 1, it indicated that the standard deviation of the positive control and the negative control was 0, and the experimental system was an ideal experimental system (Zhang, Chung, Oldenburg. A Simple Statistical Parameter for Use in Evaluation and Validation of High Throughput Screening Assays. Journal of biomolecular screening, 1999, 4(2): 67-73).

According to the concentration range of AChI to activate the reporter cell line, five AChI concentration points were selected in the range of 10 to $1 \times 10^5$ nM, and the optimal AChE content and reaction time in the AChE reaction system were investigated under these conditions.

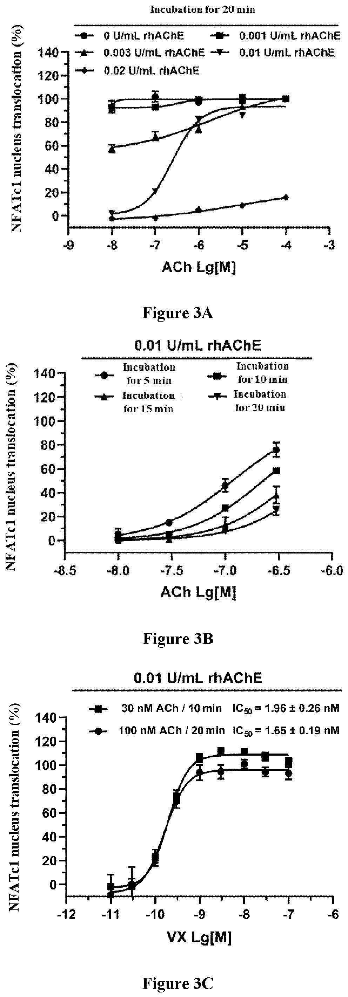

When rhAChE at 0.001, 0.003, 0.01 and 0.02 U/mL (this concentration was the initial concentration of rhAChE in the AChE reaction system, that was, the enzyme concentration in the system when it did not react with AChI) was incubated with AChI at different concentrations for 10 minutes, the results of activation of the reporter cell line by each reaction system were shown in Figure 3A. When the enzyme content was too low (≤0.003U/mL), AChI could not be effectively hydrolyzed; when the enzyme content was too high (0.02 U/mL), AChI would be excessively hydrolyzed, and there was limited difference between the two conditions in the reporter cell analysis; under the condition of 0.01 U/mL rhAChE, AChI concentration-dependently induces EGFP-NFATc1 nuclear translocation in the range of 10 to 1000 nM. The AChI in the above concentration range had nearly 100% cell activation effect in the enzyme-free system, suggesting that within 10 minutes, the amount of substrate AChI hydrolyzed by 0.01 U/mL rhAChE could be quantified by the reporter cell line. Therefore, the suitable initial concentration of rhAChE in the reaction system could be 0.005 to 0.018 U/mL, preferably 0.007 to 0.015 U/mL, and further preferably 0.01 U/mL.

Considering the feasibility of antagonist screening experiment, the initial concentration of substrate AChI in the AChE reaction system was set to 10, 30, 100, and 300 nM within the concentration-dependent range, and the effects of different time for incubation of 0.01 U/mL enzyme and substrate on the results were investigated. The results were shown in Figure 3B. At the lowest substrate concentration (10 nM), the hydrolysis was completed within 5 minutes, and the feasibility of subsequent experiment was low. At the highest substrate concentration (300 nM), the residual amount of substrate at each time point was too high and the difference was significant, so that the subsequent experiment was lack of sufficient window and stability; while in the following two cases, i.e., the substrate at the concentration of 30 nM was incubated with enzyme for 10 minutes, and the substrate at the concentration of 100 nM was incubated with enzyme for 20 minutes, the hydrolysis of AChI was greater than 90%, the system had basically reached stability. This condition not only met the requirement of the maximum window value for inhibitor screening, but also was beneficial to the stability of the system and had strong operability. Thus, it was preliminarily determined that the two reaction systems, i.e., incubation of 0.01 U/mL rhAChE and 30 nM ACh for 10 minutes, and incubation of 0.01 U/mL rhAChE and 100 nM ACh for 20 minutes, were preferred.

On this basis, the above two screening systems were further evaluated using the known acetyl esterase inhibitor VX. The results were shown in Figure 3C, in which in both reaction systems, VX could concentration-dependently increase the activity of cholinergic receptor in reporter cells, that was, inducing nuclear translocation of EGFP - N F A T c 1 in reporter cells, with the lowest detection concentration of 0.1 nM; the Z' factors of the two methods composed of the two reaction systems were 0.716 and 0.614 respectively, that was, the two reaction systems both could sensitively and reliably reflect the inhibitory activity of VX on AChE when combined with the reporter cell line, in which the Z' factor under condition of incubation of 0.01 U/mL rhAChE with 30 nM ACh for 10 minutes was higher, so this system was selected as the optimal reaction system. The method for screening cholinesterase inhibitor (AChEI) composed of this system and the reporter cell line had high-throughput detection capability, and relatively good stability and sensitivity.

Example 3: Evaluation of inhibitory activity of different types of AChEI against AChE by using the method for screening AChEI established in the present disclosure

In this example, the method established in the example of the present disclosure was used to detect the inhibition activity of organophosphorus pesticides commonly used in agricultural production such as paraoxon, dichlorvos (DDVP) and chlorpyrifos, AD therapeutic drugs such as donepezil and rivastigmine, and nerve agents such as sarin (GB), tabun (GA), soman (GD) and VX on AChE. The specific steps were as follows: To 30 $\mu$L of rhAChE working solution in each well, 30 $\mu$L of AChEI with corresponding gradient concentration was added, and the mixed solution was incubated at 37°C for 30 min to allow AChEI and rhAChE to fully interact, then 60 $\mu$L of AChI working solution was added and reacted at 37°C for 10 min. Then, 100 $\mu$L of the reaction solution was pipetted and added to a black transparent-bottom 96-well plate pre-inoculated with the reporter cell line. After incubation for 20 minutes, the activation degree of the reporter cell line was quantitatively analyzed according to the method described in Example 1 to determine the inhibition activity of AChEI against AChE.

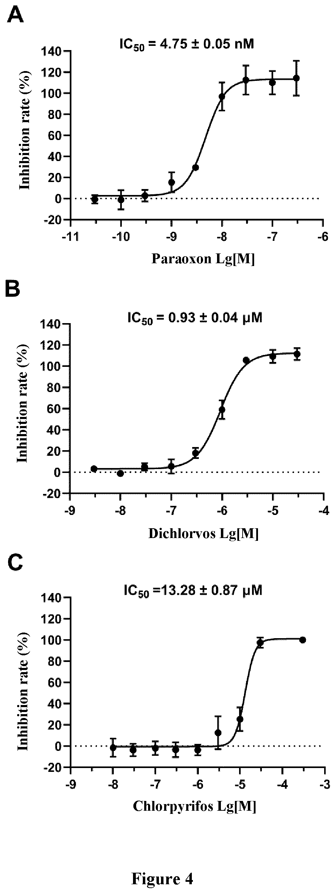

The results of the inhibition effect of organophosphorus pesticides against rhAChE activity were shown in Figure 4, in which paraoxon, dichlorvos, and chlorpyrifos all could inhibit rhAChE in the cell detection system, and reduce the hydrolysis of AChI in the system, and then activate the M3 receptor of the reporter cells, thereby promoting the EGFP-NFATc1 nuclear translocation. The lowest detection concentrations of the three organophosphorus pesticides were 1 nM, 0.3 $\mu$M, and 3 $\mu$M, respectively, and the IC$_{50}$ values were: 4.75 $\pm$ 0.05 nM, 0.93 $\pm$ 0.04 $\mu$M, and 13.28 $\pm$ 0.87 $\mu$M, respectively. It was suggested that the sequence of the inhibition activities of the three was: paraoxon > dichlorvos > chlorpyrifos, which was consistent with the reported results, indicating that the method established in the example of the present disclosure could be used to evaluate the acetylcholinesterase inhibitory activity of different OP pesticides.

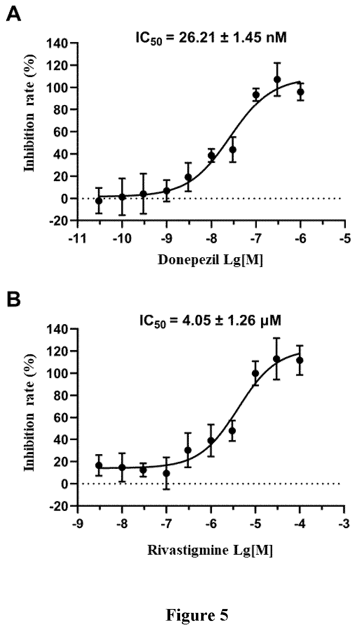

The results of the inhibition effects of donepezil and rivastigmine against rhAChE were shown in Figure 5, in which both donepezil and rivastigmine could promote the EGFP-NFATc1 nuclear translocation by inhibiting rhAChE in the cell detection system, and their IC$_{50}$ values were: 26.21 $\pm$ 1.45 nM, 4.05 $\pm$ 1.26 $\mu$M, respectively. This result was basically consistent with the results reported in the literature.

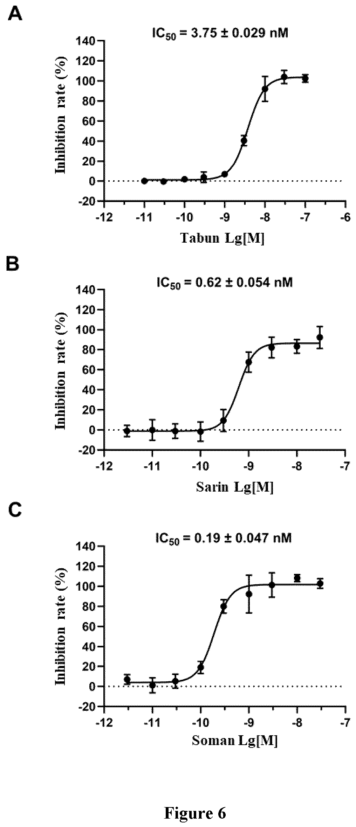

The results of the inhibition effects of OP nerve agents against rhAChE were shown in Figure 6 and Figure 3C, in which tabun, sarin, soman and VX all significantly inhibited rhAChE in the cell detection system, and promoted the EGFP-NFATc1 nuclear translocation; and their IC$_{50}$ values were: 3.75 $\pm$ 0.03 nM (GA); 0.62 $\pm$ 0.05 nM (GB); 0.19 $\pm$ 0.05 nM (GD), 0.19 $\pm$ 0.03 nM (VX). The minimum detection concentration of VX reached 30 pM. This result was consistent with the results of our laboratory using the Ellman method (in which tabun was partially degraded).

Based on the evaluation results of the above three major categories of AChEI, it was shown that the method established in the present disclosure could quantitatively screen different types of AChEI with high throughput, and this method had the characteristics of high sensitivity, strong stability, convenient operation, and low consumption.

Example 4: Application of the method for screening AChEI established in the present disclosure in characterizing phenotypic characteristics of AChEI cells

In addition to screening AChEI and detecting the enzyme inhibitory effect of AChEI, the method for screening AChEI established in the present disclosure could also be combined with fluorescent staining of nucleus and cytoskeleton (actin, tubulin) to characterize the phenotypic characteristics of the cell used to detect AChEI, which indicated the unique cellular activity and toxicity information of the substance to be tested. The specific method was as follows:
The treated cells were stained with Hoechst 33342 according to the method described in Example 1, and permeabilized by using 200 $\mu$L of 1$\times$PBS solution containing 0.3% Triton X-100 for 30 minutes at 37°C in the dark; then 633-labeled phalloidin was added at 50 $\mu$L/well for staining at 37°C for 30 min in the dark. The supernatant was discarded. The cells were washed 3 times with 1$\times$PBS solution, then 200 $\mu$L of blocking solution (PBS solution containing 5% BSA) was added to each well, and the cells were blocked at room temperature for 1 h. The blocking solution was discarded, and 50 $\mu$L of mouse anti-human $\alpha$-Tubulin monoclonal antibody as the primary antibody was added to each well, and then the cells were incubated at 37°C in the dark for 1 h. The primary antibody was recovered, the cells were washed 3 times with the blocking solution, and 50 $\mu$L of Alexa Flour 546-labeled donkey anti-mouse IgG as secondary antibody was added to each well, and the cells were incubated at room temperature for 30 min in the dark. The cells were washed twice with 1$\times$PBS solution, and 100 $\mu$L of 1$\times$PBS solution was added to the well. The high-content cell imaging and analysis system was used to acquire cell images, and analyze the status of nuclear morphology, actin, and tubulin.

Different exogenous substances have different targets, induce different cell changes, and have different cell phenotypic characteristics. There are many types of AChEIs with diverse structures and different biological effects. Studies have shown that in addition to the specific target AChE, OPs also affect other targets, such as hundreds of enzymes, receptors and other proteins, some of which may be particularly relevant to the long-term CNS effects of chronic OP exposure and developmental neurotoxicity, and AChE interactions with these targets are becoming part of the cumulative risk assessment of OPs. The method for screening AChEI established in the present disclosure could not only identify AChEI by detecting the degree of EGFP-NFATc1 nuclear translocation, but also quantitatively detect the changes in cellular and subcellular morphological structures, signaling pathways and target proteins induced by the molecules to be tested through compatible fluorescent labels in the same experiment, thereby obtaining unique cell phenotype spectrum characteristics of the molecules to be tested. This provides richer information for screening and identifying new AChEIs.

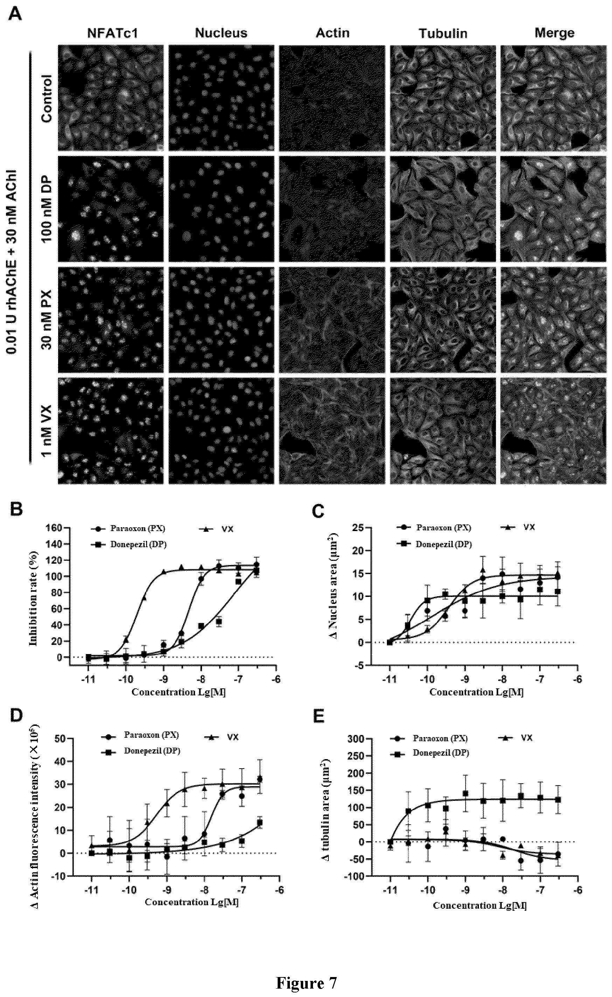

In this example, different types of AChEI (nerve agent VX, OP pesticide paraoxon, and AD drug donepezil) were used as representatives to detect EGFP-NFATc1 nuclear translocation so as to determine target-specific activity, in the same analysis procession, the changes in cell nucleus, actin and tubulin were analyzed exemplarily and quantitatively through different fluorescent labeling of channels. They can not only reflect the rapid response characteristics of cellular and subcellular morphology and cytoskeleton caused by interference of exogenous substances, but are also important indicators of toxicity and potential mechanisms. The four-parameter fluorescence imaging and quantitative results of cells treated with different types of AChEI were shown in Figure 7. As shown in the EGFP-NFATc1 nuclear translocation picture, VX, paraoxon and donepezil all could inhibit the activity of AChE in a concentration-dependent manner, but their concentration-activity curves had different ranges and slopes. The slopes of VX and paraoxon were basically the same, and the slope of donepezil was significantly smaller, suggesting that the way donepezil bound to AChE was different from those of VX and paraoxon (which was basically consistent with literature reports). Although all of the three showed increased expression level of cell actin (actin) in a concentration-dependent manner (A and B in Figure 7), their curve slopes were quite different; all of the three showed increased nuclear area (A and C in Figure 7), but their concentration effect curves were different; in addition, after the treatment of the three, their tubulin distributions in cells were obviously different (A in Figure 7), that was, starting at 1 nM, donepezil showed increased tubulin distribution in a concentration-dependent manner, while for VX and paraoxon, the cellular tubulin distribution slightly decreased as the concentration increased. These results showed that in addition to inhibiting AChE activity, the three presented their own phenotypic change characteristics; VX and paraoxon, both OPs, had more similar cell phenotypic characteristics, which were quite different from the AD drug donepezil. Accordingly, according to the purpose of screening, the phenotypic characteristics could be enriched by expanding time points and different cell phenotypes, and finally the unique phenotypic spectrum of each substance to be tested could be obtained, thereby providing more basis for screening and identification.

Although the specific embodiments of the present disclosure have been described in detail, those skilled in the art will understand that various modifications and changes can be made to the details based on all teachings that have been disclosed, and these changes are within the protection scope of the present disclosure. The entirety of the present disclosure is given by the appended claims and any equivalents thereof.

## Claims

**1.** A method for screening acetylcholinesterase inhibitor, comprising:

> 1) mixing a sample to be screened with an acetylcholinesterase;
> 2) mixing the mixed system obtained in step 1) with an acetylcholine;
> 3) contacting a reporter cell M3:NFATc1-EGFP U2OS with the mixed system obtained in step 2);
> 4) detecting the degree of activation of the reporter cell M3:NFATc1-EGFP U2OS.

**2.** The method according to claim 1, wherein the degree of activation of the reporter cell M3:NFATc1-EGFP U2OS is expressed as the degree of translocation of NFATc1-EGFP from cytoplasm to nucleus in the reporter cell,

> if the NFATc1-EGFP in the reporter cell is translocated from cytoplasm to nucleus, it is judged that the sample to be screened contains an acetylcholinesterase inhibitor,
> preferably, the inhibitory activity of acetylcholinesterase inhibitor against acetylcholinesterase is quantified according to the number of intracellular NF AT c 1- EGFP translocated from cytoplasm to nucleus.

**3.** The method according to claim 1 or 2, wherein the acetylcholine is acetylcholine iodide, and the acetylcholinesterase is a recombinant human acetylcholinesterase.

**4.** The method according to claim 1 or 2, wherein

> in step 1), the sample to be screened is mixed with the acetylcholinesterase in a first solvent;
> in step 2), the mixed system obtained in step 1) is mixed with the acetylcholine in a second solvent; the first solvent and the second solvent are the same or different, and are each independently a medium suitable for the normal growth of the reporter cell M3:NFATc1-EGFP U2OS, such as a DMEM high-glucose medium,

preferably, in step 2), after the mixed system obtained in step 1) is mixed with acetylcholine, the initial concentration of acetylcholinesterase in the obtained mixed system is 0.005 to 0.018 U/mL, preferably 0.007 to 0.015 U/mL, and further preferably 0.01 U/mL,

preferably, in step 2), after the mixed system obtained in step 1) is mixed with acetylcholine, the initial concentration of acetylcholine in the obtained mixed system is 10 to 1000 nM, preferably 30 to 800 nM, further preferably 30 to 500 nM, further preferably 30 to 400 nM, further preferably 30 to 300 nM, further preferably 30 to 200 nM, further preferably 30 to 100 nM.

4. The method according to any one of claims 1 to 3, wherein

preferably, in step 1), the sample to be screened is mixed with the acetylcholinesterase at a temperature of 25 to 40 °C (e.g., 30°C, 32°C, 35°C, 37°C) for 10 to 60 minutes, for example 20 minutes, 25 minutes, 30 minutes, 35 minutes, 40 minutes, 50 minutes,

preferably, in step 2), the mixed system obtained in step 1) is mixed with the acetylcholine at a temperature of 25 to 40°C (such as 30°C, 32°C, 35°C, 37°C) for 10 to 60 minutes, for example, 20 minutes, 25 minutes, 30 minutes, 35 minutes, 40 minutes, 50 minutes.

5. The method according to any one of claims 1 to 4, wherein in step 3), the reporter cell M3:NFATc1-EGFP U2OS is contacted with the mixed system obtained in step 2) for 10 to 60 minutes, such as 20 minutes, 25 minutes, 30 minutes, 35 minutes, 40 minutes, 50 minutes.

6. The method according to any one of claims 1 to 5, wherein before step 4), the method further comprises:

fixing the reporter cell with a formaldehyde solution diluted in a phosphate buffer;
staining (e.g., fluorescently staining) the nucleus and/or cytoskeleton (actin, tubulin) of the reporter cell.

7. The method according to any one of claims 1 to 6, wherein a high-content cell imaging and analysis system is used to detect the degree of activation of reporter cell M3:NFATc1-EGFP U2OS.

8. A kit, comprising a reporter cell M3:NFATc1-EGFP U2OS, an acetylcholine (e.g., acetylcholine iodide), an acetylcholinesterase (e.g., a recombinant human acetylcholinesterase) and a fluorescent dye (e.g., Hoechst 33342, phalloidin),

preferably, the kit further comprises a medium suitable for the growth of the reporter cell M3:NFATc1-EGFP U2OS, such as a DMEM high-glucose medium,
preferably, the kit further comprises a phosphate buffer substance (in the form of an aqueous solution or anhydrous form), and formaldehyde,
optionally, the kit further comprises an instruction describing the method according to any one of claims 1 to 7.

9. Use of the kit according to claim 8 for screening an acetylcholinesterase inhibitor.

10. A system for screening acetylcholinesterase inhibitor, comprising the kit according to claim 8 and a high-content cell imaging and analysis system.

**Figure 1**

Figure 2

Incubation for 20 min

**Figure 3A**

0.01 U/mL rhAChE

**Figure 3B**

0.01 U/mL rhAChE

**Figure 3C**

Figure 4

**A**

IC$_{50}$ = 26.21 ± 1.45 nM

Inhibition rate (%) vs Donepezil Lg[M]

**B**

IC$_{50}$ = 4.05 ± 1.26 µM

Inhibition rate (%) vs Rivastigmine Lg[M]

Figure 5

Figure 6

Figure 7

<div style="text-align: center;">

**INTERNATIONAL SEARCH REPORT**

</div>

| | International application No. |
|---|---|
| | **PCT/CN2023/071987** |

| | |
|---|---|
| **A. CLASSIFICATION OF SUBJECT MATTER** | |
| C12Q1/02(2006.01)i;C12Q1/46(2006.01)i;A61K35/12(2015.01)i | |
| According to International Patent Classification (IPC) or to both national classification and IPC | |

| **B.** | **FIELDS SEARCHED** |
|---|---|

Minimum documentation searched (classification system followed by classification symbols)

C12Q,A61K

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN, pubmed, elsevier science, 百度学术 BAIDU SCHOLAR: 报告细胞, 胆碱, 毒蕈碱, 钙, 活性, 检测, 酶, 酶抑制, 筛查, 筛选, 移位, 乙酰胆碱, 乙酰胆碱酯酶, 抑制剂, 易位, 异位, 转位, acetylcholine, acetylcholinesterase, Ach, AChE, activity, Ca, CHRM, detect, discover, GFP, inhibitor, M3, muscarinic, NFATc, NFATc1, screen, NFATc+, muscarinic receptor, inhibit+, M-R, M3/NFAT, nuclear factor of activate T cell, NFATc?

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| PX | CN 115369145 A (ACADEMY OF MILITARY MEDICAL SCIENCES OF THE PLA ACADEMY OF MILITARY SCIENCE) 22 November 2022 (2022-11-22) claims 1-11 | 1-10 |
| A | CN 102016580 A (GE HEALTHCARE UK. LTD.) 13 April 2011 (2011-04-13) see abstract, description, paragraphs 147-236, and figures 11-16 | 1-10 |
| A | CN 1865929 A (SHANGHAI INSTITUTE OF ORGANIC CHEMISTRY, CHINESE ACADEMY OF SCIENCES) 22 November 2006 (2006-11-22) see abstract, and embodiments 1-4 | 1-10 |
| A | US 2021371899 A1 (ACADEMIA SINICA) 02 December 2021 (2021-12-02) see abstract, claims 1-6, and embodiments | 1-10 |

| ☑ Further documents are listed in the continuation of Box C. | ☑ See patent family annex. |
|---|---|

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 April 2023** | **07 May 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/071987** |

**C.    DOCUMENTS CONSIDERED TO BE RELEVANT**

| Category* | Citation of document, with indication, where appropriate, of the relevant passages | Relevant to claim No. |
|---|---|---|
| A | 冯帆 (FENG, Fan). "药物/候选药物细胞表型谱研究 (Cell imagine phenotype Profiles of drugs and candidate drugs)" <br> *中国博士学位论文全文数据库医药卫生科技辑 (China Doctoral Dissertations Full-text Database, Medicine and Health Sciences)*, No. 11, 15 November 2013 (2013-11-15), E079-22 <br> see abstract, page 12 paragraph 1-page 15 last paragraph, page 91 paragraph 1, and page 171 paragraph 4, and table 2 | 1-10 |
| A | 雷春阳 (LEI, Chunyang). "超电荷绿色荧光蛋白在生化传感中的研究与应用 (The Research and Application of Supercharged Green Fluorescent Protein in Biosensing)" <br> *中国博士学位论文全文数据库 工程科技I辑 (China Doctoral Dissertations Full-text Database; Engineering Science and Technology I)*, No. 9, 15 September 2015 (2015-09-15), <br> see abstract, page 73 paragraph 3- last paragraph, page 83 paragraph 1-page 86 paragraph 1 | 1-10 |
| A | ZHENG, Qingqing et al. "Protein-mimicking nanowire-inspired electro-catalytic biosensor for probing acetylcholinesterase activity and its inhibitors" <br> *Talanta*, Vol. 183, 09 February 2018 (2018-02-09), 258–267 <br> see abstract, and flow 1, page 259, left column, paragraph 1 to page 260, right column, and paragraph 3 | 1-10 |

Form PCT/ISA/210 (second sheet) (July 2022)

**INTERNATIONAL SEARCH REPORT**
**Information on patent family members**

International application No.

**PCT/CN2023/071987**

| Patent document cited in search report | | | Publication date (day/month/year) | Patent family member(s) | | | Publication date (day/month/year) |
|---|---|---|---|---|---|---|---|
| CN | 115369145 | A | 22 November 2022 | None | | | |
| CN | 102016580 | A | 13 April 2011 | WO | 2009090215 | A1 | 23 July 2009 |
| | | | | GB | 0800938 | D0 | 27 February 2008 |
| | | | | JP | 2011510282 | A | 31 March 2011 |
| | | | | JP | 5574979 | B2 | 20 August 2014 |
| | | | | US | 2014199704 | A1 | 17 July 2014 |
| | | | | EP | 2255186 | A1 | 01 December 2010 |
| | | | | EP | 2255186 | B1 | 23 March 2016 |
| | | | | US | 2010311069 | A1 | 09 December 2010 |
| | | | | US | 8673554 | B2 | 18 March 2014 |
| CN | 1865929 | A | 22 November 2006 | None | | | |
| US | 2021371899 | A1 | 02 December 2021 | EP | 3867362 | A1 | 25 August 2021 |
| | | | | EP | 3867362 | A4 | 03 August 2022 |
| | | | | TW | 202033961 | A | 16 September 2020 |
| | | | | TWI | 744705 | B | 01 November 2021 |
| | | | | WO | 2020081765 | A1 | 23 April 2020 |
| | | | | JP | 2022501015 | A | 06 January 2022 |
| | | | | JP | 7231714 | B2 | 01 March 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210059880

**Non-patent literature cited in the description**

- **SAMBROOK J et al.** Molecular Cloning: A Laboratory Manual. Cold Spring Harbor Laboratory Press, 1989
- **F. M. AUSUBEL et al.** Short Protocols in Molecular Biology. John Wiley & Sons, Inc., 1995
- **ZHANG** ; **CHUNG** ; **OLDENBURG**. A Simple Statistical Parameter for Use in Evaluation and Validation of High Throughput Screening Assays. *Journal of biomolecular screening*, 1999, vol. 4 (2), 67-73